# EUROPEAN PATENT APPLICATION

(11) **EP 3 037 826 A1**
(43) Date of publication of application: **29.06.2016**
(21) Application number: 13891817.2
(22) Date of filing: 23.08.2013
(51) Int. Cl.: G01N 35/08, B01J 19/00, B81B 1/00, B81C 3/00, G01N 37/00

(54) **MICROCHEMICAL CHIP AND REACTION DEVICE**

(71) Applicant: ASAHI FR R&D Co., Ltd., Saitama-shi, Saitama 330-0801 (JP)
(72) Inventor: TAKAGI, Kazuhisa, Saitama-shi Saitama 330-0801 (JP); TAKANO, Tsutomu, Saitama-shi Saitama 330-0801 (JP)
(74) Representative: dompatent von Kreisler Selting Werner - Partnerschaft von Patent- und Rechtsanwälten mbB
(86) International application number: PCT/JP2013/072590
(87) International publication number: WO 2015/025424

(57) **Abstract**

Provided is a simple and compact microchemical chip which has a fine flow path reliably formed therein through which a valuable specimen originated from a biological object or a trace amount of a dilute reagent is made to flow; does not break even when a fluid sample is made to flow through pressurization; makes it possible to flow the fluid sample to the flow path accurately, reliably, and as desired; makes it possible to accurately, simply, and quickly analyze a useful substance such as a biological component of the specimen and cause the useful substance to react; and can be produced with a high yield, on a large scale, and so as to be homogeneous. A microchemical chip 1 comprises: a rubber sheet 20 having a penetrated flow path 26 which chemically reacts a pressurized fluid sample selected from a specimen and a reagent by flowing thereinto; substrate sheets 10,30 which sandwich the rubber sheet 20 and bond to both faces thereof by direct bond or by chemical bond through a silane-coupling agent and are selected from the group consisting of metal, ceramics, glass, and resin; and a hole for injecting the fluid sample 11a, 11b into the flow path and a hole for draining the fluid sample flowed therefrom 12a, 12b, 12c which are opened into the substrate sheet 10.

## Description

### Technical Field

The present invention relates to a microchemical chip which is used by being installed to an analysis apparatus used in microanalysis of a biological component included into a test sample which is a specimen originated from a biological object, or a reaction device such as a microreactor used in chemical microsynthesis of a useful substance such as the biological component etc. exhibiting pharmacological effect.

### Background of the Art

In order to quantitate a reaction amount of an enzyme which acts on a substrate in a specimen or an amount of the substrate by degree of color generation depending on a reagent which is colored by the enzyme or substrate, a microbiological chip has been used. In this regard, specific substrate selectivity of the enzyme is utilized by using a trace amount like µl order of a test sample such as blood, urine, and the like, which are the specimen originated from a biological object. Further, when a quantitative analysis of the substrate amount by converting the enzyme reaction amount into an electric signal by using a membrane including the enzyme and electrodes, DNA extraction and a polymerase chain reaction (PCR) amplification thereof, or ion concentration measurement, microsynthesis etc. of nucleic acid, saccharide, protein, or peptide is conducted in µM order, a microreactor chip has been used.

A microchemical chip such as the microbiological chip and the microreactor has a channel-shaped micro flow path as a reaction channel which mixes, reacts, separates, and detects the specimen and/or the reagent which are pressurized, imported, and flowed therein. According to the conventional microchemical chip, the micro flow path of several dozen to several hundreds of micrometers is formed into an inorganic substrate such as a stainless substrate, silicon substrate, quartz substrate, and glass substrate, and an organic substrate of a resin substrate or a rubber substrate by means of cutting or etching.

The microchemical chip made of the stainless substrate, the silicon substrate, or quartz substrate is difficult to produce on a large scale, is expensive, and lacks generalities, because it is difficult to conduct cutting work to form the micro flow path due to hardness of raw material thereof, while the substrates bonded therebetween are difficult to deform. The microchemical chip made of the glass substrate must be produced through steps of: serially applying metal such as chromium and photoresist on a surface of the raw material of the glass substrate; printing a pattern of a micro channel by exposing the photoresist; developing the photoresist; chemically etching by using hydrofluoric acid; and removing the photoresist. Thus, the cumbersome steps having laborious processes, causes difficulty to accurately form the micro flow path, and are not suitable for producing on the large scale.

On the other hand, Patent Document 1 discloses that a microchemical chip made of the organic substrate is formed from a high-transparent plastic resin. Because the resin substrate and a rubber substrate are easy to be shaped or cut, the microchemical chip, which is formed by adhering these substrates through adhesive agent or by heat sealing, is suitable for producing on the large scale. Especially, the microchemical chip made of the transparent resin substrate is useful to an optical system analysis.

Because the organic substrate is difficult to deteriorate against a water soluble specimen and/or reagents such as strong acids of hydrofluoric acid etc. which solve a metal or water soluble chemical agent, the microchemical chip made of the organic substrate is chemically stable. But, because a resin sheet or rubber sheet having the formed flow path is adhered through the adhesive agent or sealed by heating, the microchemical chip has low bonding strength. Therefore, when the high-pressurized specimen and/or reagent flows in the flow path, the bonded substrates cannot withstand the pressure and break up adhesion therebetween and thus, the microchemical chip is physically weak and is easily broken. Even if the specimen and/or the reagent is pressurized by low pressure so as to avoid breakdown of the microchemical chip in order to flow the specimen or reagent into the fine, branched, and complex-patterned flow path, the specimen or reagent is difficult to reach the terminal thereof. Because interposition of the adhesive agent or overheating causes outflow of the adhesive agent to the flow path, fluctuation of a refractive index, or heat deforming and distortion, the microchemical chip made of the transparent resin sheets having the flow path bonded by adhering through the adhesive agent or by heat sealing is difficult to have homogeneous transparency, which is important to the fine optical system analysis, on the flow path.

### Prior Art Document

### [Patent Document]

[Patent Document 1] Japanese Patent Application Publication No. 2006-218611

### Summary of the Invention

### Problems to be Solved by the Invention

The present invention was made in view of solving the above described problems, and its object is to provide a simple and compact microchemical chip which has a fine flow path reliably formed therein through which a valuable slight specimen originated from a biological object and/or a trace amount of a dilute reagent is made to flow; does not break even when a fluid sample is made to flow through pressurization under low to high temperature; makes it possible to flow the fluid sample to the flow path accurately, reliably, and as desired; makes it possible to accurately, simply, and quickly analyze a useful substance such as a biological component of the specimen and cause the useful substance to react; and can be produced with a high yield, on a large scale, and so as to be homogeneous. And another object is to provide a reaction device for using thereof.

### Means for Solving Problems

A microchemical chip developed to achieve the objects above described comprises: a rubber sheet having a penetrated flow path which chemically reacts a pressurized fluid sample selected from a specimen and a reagent by flowing thereinto; substrate sheets which sandwich the rubber sheet and bond to both faces thereof by direct bond or by chemical bond through a silane-coupling agent and are selected from the group consisting of metal, ceramics, glass, and resin; and a hole for injecting the fluid sample into the flow path and a hole for draining the fluid sample flowed therefrom which are opened into the substrate sheet.

In the microchemical chip of claim 2 according to claim 1, the rubber sheet and the substrate sheets are bonded by the chemical bond which is formed under conditions of reduced pressure and/or pressurization.

In the microchemical chip, the rubber sheet and the substrate sheets are bonded by the chemical bond which is formed under conditions of pressurization and/or heating after reduced pressure condition.

In the microchemical chip, the rubber sheet and/or the substrate sheet are given an active treatment at these bonded faces.

In the microchemical chip, a plurality of the rubber sheets which are sandwiched between the substrate sheets is composed by stacking thereof.

In the microchemical chip, the outermost substrate sheets are sandwiched between plate-shaped holders, and fixed with the rubber sheet so as to inhibit leakage of the fluid sample.

In the microchemical chip, the rubber sheet is made from silicone rubber.

In the microchemical chip, the rubber sheet which is made from silicone rubber and the substrate sheets are activated by a corona discharge treatment, a plasma treatment and/or an ultraviolet irradiation treatment over at least any one of these bonded faces, and bonded by the direct bond.

In the microchemical chip, the rubber sheet which is made from silicone rubber or non-silicone rubber and the substrate sheets are activated by a corona discharge treatment, a plasma treatment and/or an ultraviolet irradiation treatment over at least any one of these bonded faces, and bonded by the chemical bond through the silane-coupling agent having an amino group and/or an alkoxy group.

In the microchemical chip, the substrate sheets are made from the resin which is at least one selected from the group consisting of a polycarbonate resin, a cycloolefin resin, a polyethylene terephthalate resin, an acryl resin, and an epoxy resin; the silane-coupling agent has the amino group and the alkoxy group.

In the microchemical, at least a side surface of the flow path of the rubber sheet is coated by coating.

A method for producing a microchemical chip comprises the steps of: a flow path forming step of forming a flow path, which reacts a pressurized fluid sample selected form a specimen and a reagent by flowing thereinto, into a rubber sheet by penetrating it; a perforating step of forming a hole for injecting the fluid sample into the flow path and a hole for draining the fluid sample flowed therefrom into the substrate sheet selected from the group consisting of metal, ceramics, glass, and resin; and a bonding step of bonding the substrate sheets to both faces of the rubber sheet by direct bond or by chemical bond through a silane-coupling agent while sandwiching the rubber sheet therebetween.

The method for producing the microchemical chip, the rubber sheet is bonded to the substrate sheets by the chemical bond under reduced pressure.

A reaction device comprises: a microchemical chip comprising a rubber sheet having a penetrated flow path which chemically reacts a pressurized fluid sample selected from a specimen and a reagent by flowing thereinto; substrate sheets which sandwich the rubber sheet, and bond to both faces thereof by direct bond or by chemical bond through a silane-coupling agent and are selected from the group consisting of metal, ceramics, glass, and resin; and a hole for injecting the fluid sample into the flow path and a hole for draining the flowed fluid sample therefrom which are opened into the substrate sheet; a pressurizer, which is connected to the hole for injecting the fluid sample, for flowing the fluid sample into the flow path by pressing it after injecting it; and a device body for installing the microchemical chip.

### Effects of the Invention

In the microchemical chip of the present invention, the rubber sheet and the substrate sheets are reliably bonded at bonded faces thereof by strong adhesion based on the direct bond or the interposing chemical intermolecular bond through a single molecular of the silane-coupling agent except an area of the flow path. Therefore, the fine flow path, which does not leak a trace amount of the valuable specimen originated from a biological object and/or a trace amount of the dilute reagent and makes it flow by pressurization, is reliably formed.

In the microchemical chip, the fine flow path from 0.5µm to 5mm in width having a linear shape combined with a straight line and a curved line, or a complex pattern shape, which is expanded, focused, or branched at the terminal or half way thereof, is accurately formed into the rubber sheet. In spite of having such fine flow path, when the fluid sample of the specimen and/or the reagent, is imported into the flow path by pressurization, and is flowed therein, the rubber sheet and the substrate sheets do not break up adhesions therebetween and thus, the microchemical chip does not break.

In the microchemical chip, even if the liquid or gaseous specimen and/or a fluent material as the reagent are imported into the fine flow path by pressurization from normal atmospheric pressure to about 5 atmospheric pressure, or at a low to high temperature range from freezing temperature or below to 80°C, generally at 20 to 80°C while repeating heating and cooling, the flow path does not break due to elasticity of the rubber sheet.

According to the microchemical chip, the specimen and/or reagent can be reliably and accurately imported into the desired flow path. In the result, the microchemical chip can precisely and simply analyze and react a useful substance such as a biological component etc. in the specimen on a short period.

The microchemical chip inhibits contact between the specimen and the rubber sheet as far as possible due to the fine flow path of the rubber sheet, and can prevent contamination and adsorption of the specimen and/or the reagent.

When the microchemical chip is used and thrown away, pollution could not occur by contamination of the other specimen or reagent, and thus it can achieve reliable results.

The microchemical chip has a simple component and an external shape of square of several millimeters to several dozen of centimeters having an extremely compact size. The microchemical chip includes the numerous and serial, parallel, or branched flow path in spite of the compact size, and may have injection openings and drain openings. Thus, the microchemical chip may give numerous functions so that plural reactions are conducted through many processes in series or parallel. Therefore, by using a portable analytical apparatus without a large-scaled analysis apparatus, a plurality of qualitative or quantitative analyses can be swiftly conducted at not only inside but also outside. Further, an amount of an analytical reagent and a reactive reagent used in the microchemical chip can be restrained to be a small amount. In addition, since an amount of a waste fluid is much more less than an analysis or reaction by using a flask or test tube, the microchemical chip helps against environmental protection.

According to the method for producing the microchemical chip, the fine flow path can be formed into the rubber sheet by using a simple process such as laser beam machining without developing and etching of photoresist. The rubber sheet and substrate sheets simply and much more strongly bonded than adhesion depending on the adhesive agent, because a chemical intermolecular bond of an ether bond is directly formed by contact between the rubber sheet and the substrate sheets except area of the flow path; an interposing covalent bond through the single molecule of the silane-coupling agent, which is applied, sprayed, or dipped, is formed therebetween; and a chemical intermolecular bond which is interaction based on a hydrogen bond and/or an electrostatic attractive force is formed therebetween. Such molecular adhesion does not need heating of high temperature as heat sealing of a thermoplastic resin, and may be formed enough by heating below the heat sealing temperature thereof for short time. Therefore, the fluctuation of the refractive index, or heat deforming and distortion which inhibit accuracy of the optical analysis does not occur.

The method therefor is extremely simple and has short processes, and the microchemical chip is produced with high quality, homogeneity, low cost, and high yield on the large scale.

According to the reaction device of the present invention, by using the microchemical chip installed to the device body, the microanalysis and/or microsynthesis of the useful substance such as the biological component etc., which is contained in the valuable specimen originated from the trace amount of the biological object and/or the trace amount of the dilute reagent, can be precisely and simply conducted on the short period.

### Brief Description of Drawings

Fig. 1 is a schematic perspective view showing a procedure for producing the microchemical chip of the present invention.
Fig. 2 is a schematic perspective view showing a procedure for producing the other microchemical chip of the present invention.
Fig. 3 is a perspective view showing a state in which the microchemical chip of the present invention is used.
Fig. 4 is a schematic perspective view showing a procedure for producing the other microchemical chip of Example 3 of the present invention.

### Mode for Carrying out the Invention

Hereunder, embodiments to practice the present invention will be explained in detail, but the scope of the present invention is not restricted by these embodiments.

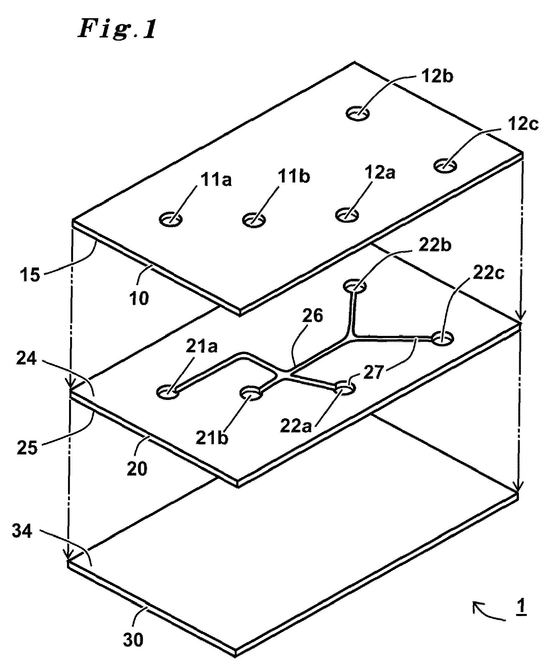

According to Fig.1 showing a procedure for producing a microchemical chip 1 of the present invention as an embodiment, the microchemical chip 1 is provided with a rubber sheet 20 stacked between a metal substrate sheet 10 for covering and a metal substrate sheet 30 for supporting a bottom face, and has flexible properties.

A channel-shaped flow path 26 is formed into the rubber sheet 20 by penetrating the both faces thereof. A fluid sample of a liquid or gaseous specimen and/or reagent is pressurized to flow into the flow path 26, and chemically is reacted thereat. The flow path 26 is extended from fluid sample injection parts 21a, 21b of starting point terminals; joined at a downstream of these parts; divided into a branch channel which extends therefrom to a fluid sample drain part 22a and a main channel which extends therefrom to fluid sample drain parts 22b, 22c; and divided in a downstream of the main channel so as to extend to the fluid sample drain parts 22b, 22c of ending point terminals. Surfaces of an upper face 24 and a lower face 25 of the rubber sheet 20 are activated except area of the flow path 26.

Fluid sample injection holes 11a, 11b and fluid sample drain holes 12a, 12b, 12c are opened into the substrate sheet 10 for covering having the same size as the rubber sheet 20 and overlapping thereon. The fluid sample injection holes 11a, 11b and the fluid sample drain holes 12a, 12b, 12c are positioned so as to correspond to the fluid sample injection parts 21a, 21b and the fluid sample drain part 22a, 22b, 22c, respectively. A surface of the lower face 15 of substrate sheet 10 for covering, which is directed to the rubber sheet 20, is activated except area of the fluid sample injection holes 11a, 11b and the fluid sample drain holes 12a, 12b, 12c.

A whole surface of an upper face 34 of the substrate sheet 30 for supporting the bottom face, which is directed to the rubber sheet 20, is activated.

Between active groups such as hydroxy groups, which are produced by activation or are originally existed, generate ether bonds as strong covalent bonds by dehydration, or new covalent bonds though a plural of functional groups in molecules of a silane-coupling agent. Therefore between these sheets 10, 20, 30 are chemically and directly bonded through the active groups.

The rubber sheet 20 may be made from any one of non-silicone rubber other than the silicone rubber. Particularly, the rubber sheet 20 may be made from the silicone rubber exemplified by a peroxide crosslinking type silicone rubber, an addition crosslinking type silicone rubber, and a condensation crosslinking type silicone rubber; a three-dimensional silicone rubber exemplified by a blended rubber of such silicone rubber mentioned above with an olefin rubber; or a non-silicone rubber. The rubber sheet 20 may be a silicone rubber elastic sheet, which is made from these rubbers by molding or stretching, and optionally crosslinking. These rubber raw materials have a number average molecular weight from ten thousand to one million.

The peroxide crosslinking type silicone rubber, which is a raw material for the rubber sheet 20, is not specifically limited as far as the rubber synthesized from a silicone raw compound and crosslinked by a peroxide type crosslinking agent. Particularly, polydimethylsiloxane, vinylmethylsiloxane/polydimethylsiloxane copolymer, vinyl-terminated polydimethylsiloxane, vinyl-terminated diphenylsiloxane/polydimethylsiloxane copolymer, vinyl-terminated diethylsiloxane/polydimethylsiloxane copolymer, vinyl-terminated trifluoropropylmethylsiloxane/polydimethylsiloxane copolymer, vinyl-terminated polyphenylmethylsiloxane, vinylmethylsiloxane/dimethylsiloxane copolymer, trimethylsiloxane group-terminated dimethylsiloxane/vinylmethylsiloxane copolymer, trimethylsiloxane group-terminated dimethylsiloxane/vinylmethylsiloxane/diphenylsiloxane copolymer, trimethylsiloxane group-terminated dimethylsiloxane/vinylmethylsiloxane/ditrifluoropropylmethylsiloxane copolymer, trimethylsiloxane group-terminated polyvinylmethylsyloxane, methacryloxypropyl group-terminated polydimethylsiloxane, acryloxypropyl group-terminated polydimethylsiloxane, (methacryloxypropyl)methylsiloxane/dimethylsiloxane copolymer, and (acryloxypropyl)methylsiloxane/dimethylsiloxane copolymer can be exemplified.

As the peroxide type crosslinking agent which is coexisted therewith, for example, ketone peroxides, diacyl peroxides, hydroperoxides, dialkylperoxides, peroxyketals, alkylperesters, percarbonates can be exemplified. More particularly, ketoneperoxide, peroxyketal, hydroperoxide, dialkylperoxide, peroxycarbonate, peroxyester, benzoylperoxide, dicumylperoxide, dibenzoylperoxide, t-butylhydroperoxide, di-t-butylhydroperoxide, di(dicyclobenzoyl)peroxide, 2,5-dimethyl-2,5-bis(t-butylperoxy)hexane, 2,5-dimethyl-2,5-bis(t-butylperoxy)hexyne, benzophenone, Michler's ketone, dimethylaminobenzoic acid ethyl ester, and benzoin ethyl ether can be exemplified.

The amount to be used as the peroxide type crosslinking agent can be arbitrarily determined depending on properties of the silane-coupling agent which is optionally used, and properties of the rubber sheet 20 which is made from the silicone rubber or kinds of the silicone rubber prepared. As the peroxide type crosslinking agent, 0.01 to 10 parts by mass, more preferably 0.1 to 2 parts by mass based on 100 parts by mass of silicone rubber can be preferably used. If the amount is less than this range, crosslink density is excessively low to give undesired properties as a silicone rubber. On the contrary, if the amount is more than this range, crosslink density is excessively high, desired elasticity cannot be obtained.

The addition type silicone rubber which is a raw material for the rubber sheet 20 can be obtained by synthesis in the presence of Pt catalyst using below composition. The composition comprises polysiloxanes having a vinyl group such as vinylmethylsiloxane/polydimethylsiloxane copolymer, vinyl-terminated polydimethylsiloxane, vinyl-terminated diphenylsiloxane/polydimethylsiloxane copolymer, vinyl-terminated diethylsiloxane/polydimethylsiloxane copolymer, vinyl-terminated trifluoropropylmethylsiloxane/polydimethylsiloxane copolymer, vinyl terminated polyphenylmethylsiloxane, vinylmethylsiloxane/dimethylsiloxane copolymer, trimethylsiloxane group-terminated dimethylsiloxane/vinylmethylsiloxane/diphenylsiloxane copolymer, trimethylsiloxane group-terminated dimethylsiloxane/ vinylmethylsiloxane/ditrifluoropropylmethylsiloxane copolymer, trimethylsiloxane group-terminated polyvinylmethylsiloxane etc.; and H group-containing polysiloxanes exemplified by H-terminated polysiloxane, methyl H siloxane/dimethylsiloxane copolymer, polymethyl H siloxane, polyethyl H siloxane, H-terminated polyphenyl(dimethyl H siloxy)siloxane, methyl H siloxane/phenylmethylsiloxane copolymer, methyl H siloxane/octylmethylsiloxane copolymer etc. The other composition for preparing the addition type silicone rubber comprises amino group-containing polysiloxanes exemplified by aminopropyl-terminated polydimethylsiloxane, aminopropylmethylsiloxane/dimethylsiloxane copolymer, aminoethylaminoisobutylmethylsiloxane/dimethylsiloxane copolymer, aminoethylaminopropylmethoxysiloxane/dimethylsiloxane copolymer, dimethylamino-terminated polydimethylsiloxane; and epoxy group-containing polysiloxanes exemplified by epoxypropyl-terminated polydimethylsiloxane, (epoxycyclohexylethyl)methylsiloxane/dimethylsiloxane copolymer, acid anhydride group-containing polysiloxanes exemplified by succinic acid anhydride-terminated polydimethylsiloxane, or isocyanato group-containing compounds such as toluyldiisocyanate, 1,6-hexamethylene diisocyanate and the like.

Processing conditions to prepare the rubber sheet 20 from these compositions cannot be determined unambiguously because the processing conditions vary with the kinds and characteristics of addition reactions, but generally the preparation can be carried out at 0 to 200°C for 1 minute to 24 hours. Under these conditions, the addition type silicone rubber can be obtained as the rubber sheet 20. In cases where preparation is carried out at a low temperature to obtain a silicone rubber having good physical properties, the reaction time should be lengthened. In cases where productivity is more emphasized rather than the physical properties, the preparation should be carried out at a higher temperature for a shorter period of time. If the preparation should be carried out within a certain period of time in compliance with the production processes or working conditions, the preparation should be carried out at a comparatively higher temperature within the range to meet a desired period of processing time.

The condensation type silicone rubber of material for the rubber sheet 20 can be obtained by synthesis in the presence of Tin catalyst using below composition. The composition is exemplified by a composition of a homocondensation component consisting of silanol group-terminated polysiloxanes exemplified by silanol-terminated polydimethyl siloxane, silanol-terminated polydiphenylsiloxane, silanol-terminated polytrifluoromethylsiloxan, silanol-terminated diphenyl siloxane/dimethylsiloxane copolymer etc.;
another composition consisting of these silanol group-terminated polysiloxanes, and crosslinking agents exemplified by tetraacetoxysilane, triacetoxymethylsilane, di t-butoxydiacetoxysilane, vinyltriacetoxysilane, tetraethoxysilane, triethoxymethylsilane, bis(triethoxysilyl)ethane, tetra-n-propoxysilane, vinyltrimethoxysilane, methyltris(methylethylketoxim)silane, vinyltris(methylethylketoximino)silane, vinyltriisopropenoxysilane, triacetoxymethylsilane, tri(ethylmethyl)oximmethylsilane, bis(N-methylbenzoamido)ethoxymethylsilane, tris(cyclohexylamino)methylsilane, triacetoamidomethylsilane, tridimethylamino methylsilane; or
another composition which is obtained from these silanol group-terminated polysiloxanes, and terminal-blocked polysiloxanes exemplified by chloro-terminated polydimethylsiloxane, diacetoxymethyl-terminated polydimethylsiloxane, terminal-blocked polysiloxane.

Processing conditions to prepare the condensation type silicone rubbers from these compositions cannot be determined unambiguously because the processing conditions vary according to the kinds and characteristics of condensation reactions, but generally the preparation can be carried out at 0 to 100°C for 10min. to 24 hours. Under these conditions, the condensation type silicone rubbers can be obtained as the rubber sheet 20. In cases where the preparation is carried out at a low temperature to obtain a silicone rubber having good physical properties, the reaction time should be lengthened. In cases where productivity is more emphasized rather than the physical properties, the preparation should be carried out at a higher temperature for a shorter period of time. If the preparation should be carried out within a certain period of time in compliance with production processes or working conditions, the preparation should be carried out at a comparatively higher temperature within the range to meet a desired period of processing time.

The blended rubber material for the rubber sheet 20 comprises the silicone rubber with the olefin rubber. As the olefin rubber, 1, 4-cis-butadiene rubber, isoprene rubber, styrene-butadiene copolymer rubber, polybutene rubber, polyisobutylene rubber, ethylene-propylene rubber, ethylene-propylene-diene rubber, chlorinated ethylene-propylene rubber, chlorinated butyl rubber can be exemplified.

As the raw material of non-silicone rubber for the rubber sheet 20, which is crosslinked by using a mixture of a raw material rubber-like substance, natural rubber, 1,4-cis butadiene rubber, isoprene rubber, polychloroprene, styrene-butadiene copolymer rubber, hydrogenated styrene-butadiene copolymer rubber, acrylonitrile-butadiene copolymer rubber, hydrogenated acrylonitrile-butadiene copolymer rubber, polybutene rubber, polyisobutylene rubber, ethylene-propylene rubber, ethylene-propylene-diene rubber, ethylene oxide-epichlorohydrin copolymer rubber, chlorinated polyethylene rubber, chlorosulfonated polyethylene rubber, alkylated chlorosulfonated-polyethylene rubber, chloroprene rubber, chlorinated acryl rubber, brominated acryl rubber, fluoro rubber, epichlorohydrin rubber and its copolymer rubber, chlorinated ethylene propylene rubber, chlorinated butyl rubber, brominated butyl rubber, homopolymer rubber or two- or three- dimensional co- or ter- polymer rubber using such a monomer as tetrafluoroethylene, hexafluoropropylene, vinylidene fluoride and tetrafluoroethylene, ethylene/tetrafluoroethylene copolymer rubber, propylene/tetrafluoroethylene copolymer rubber, ethylene-acryl rubber, epoxy rubber, urethane rubber, liner polymer of both terminals unsaturated-group elastomer etc. can be exemplified. These rubbers may be used solely or mixture thereof.

The preferred raw material of the rubber sheet 20 is especially the silicone rubber.

The flow path 26 of the rubber sheet 20 may have 0.5µm to 5mm, preferably 10 to 1000µm in width, the especially non-restricted shape, any one of a straight line and curved line having a continuous liner shape and/or branched linear shape, and an arrangement of a singular or plural parallel. The rubber sheet 20 preferably has 5 to 100µm in the thickness. Since the flow path 26 has the narrow width and the rubber sheet 20 has the thin thickness, a contact area between the specimen and/or the reagent and the rubber sheet may be minimalized. Further, contamination of the specimen and/or the reagent due to leakage of a rubber component from the rubber sheet and an adsorption thereof to the rubber component may be prevented. In order to prevent the contamination and adsorption of the specimen and/or the reagent, when at least a side surface 27 of the flow path 26 of the rubber sheet 20 is coated or deposited with a non-reactive resin exemplified by a fluorine resin such as a polytetrafluoroethylene resin, a phosphoric resin such as 2-(methacryloyloxy)ethyl 2-(trimethylammonio)ethyl phosphate (MPC) polymer, and a paraxylylene resin such as parylene; or is deposited with a non-reactive inorganic substance such as titanium dioxide and silicon dioxide, the contamination and adsorption of the specimen and/or reagent may be more prevented completely to avoid contact between the rubber sheet and the specimen and/or the reagent.

The substrate sheets 10, 30 are made from a ceramics, glass, or resin other than the metal; may be formed so as to have a single plate-shaped or thin-layered shape; and may be worked by lamination. Although the substrate sheets 10, 30 have comparative stability against the specimen or the reagent, portions of the substrate sheets 10, 30, which contact to the specimen or reagent, are preferably made from the resin, coated thereby, or formed by the lamination.

As the metal which is the material for the substrate sheets 10, 30, metal such as gold, silver, copper, iron, cobalt, silicon, lead, manganese, tungsten, tantalum, platinum, cadmium, tin, palladium, nickel, chromium, titanium, zinc, aluminum, magnesium and a binary-, ternary- and multi-component metal alloys comprising of those metals can be exemplified.

As the ceramics which is the material for the substrate sheets 10,30, oxide, nitride, and carbide of metal such as silver, copper, iron, cobalt, silicon, lead, manganese, tungsten, tantalum, platinum, cadmium, tin, palladium, nickel, chromium, indium, titanium, zinc, calcium, barium, aluminum, magnesium, sodium, potassium etc., and a single or composite body thereof can be exemplified.

As the glass, which is the material for the substrate sheets 10, 30, quartz, borosilicate glass, and non-alkaline glass can be exemplified.

As the resin which is the material for the substrate sheet 10, 30, a resin such as polycarbonate resin, cycloolefin resin, acryl resin, epoxy resin, polyethylene terephthalate resin, polybutylene terephthalate resin, cellulose and derivatives thereof, hydroxyethyl cellulose, starch, diacetylcellulose, surface-saponified vinylacetate resin, low-density polyethylene, high-density polyethylene, i-polypropylene, petroleum resin, polystyrene, s-polystyrene, chromane-indene resin, terpene resin, styrene-divinylbenzene copolymer, ABS resin, polymethyl acrylate, polyethyl acrylate, polyacrylonitrile, polymethyl methacrylate, polyethyl methacrylate, polycyanoacrylate, polyvinyl acetate, polyvinyl alcohol, polyvinylformal, polyvinylacetal, polyvinyl chloride, vinyl chloride-vinyl acetate copolymer, vinyl chloride-ethylene copolymer, polyvinylidene fluoride, vinylidene fluoride-ethylene copolymer, vinylidene fluoride-propylene copolymer, 1,4-trans-polybutadiene, polyoxymethylene, polyethylene glycol, polypropylene glycol, phenol-formalin resin, cresol-formalin resin, resorcin resin, melamine resin, xylene resin, toluene resin, glyptal resin, modified glyptal resin, unsaturated polyester resin, allylester resin, 6-nylon, 6,6-nylon, 6,10-nylon, polyimide, polyamide, polybenzimidazole, polyamideimide, silicon resin, silicone rubber, silicone resin, furan resin, polyurethane resin, polyphenyleneoxide, polydimethylphenyleneoxide, mixture of triallyl isocyanurate compound with polyphenyleneoxide or polydimethylphenyleneoxide, mixture of (polyphenyleneoxide or polydimethylphenyleneoxide, triallyl isocyanurate, peroxide), polyxylene, polyphenylenesulfide (PPS), polysulfone (PSF), polyethersulfone (PES), polyether ether ketone (PEEK), polyimide (PPI, Kapton), polytetrafluroethylene (PTFE), liquid crystal resin, Kevlar fiber, carbon fiber, polymeric material exemplified by a mixture of a plurality of these resins, and crosslinked products thereof can be exemplified.

When bonded faces between the substrate sheet 10, 30 and the rubber sheet 20 are activated by means of artificiality, for example a corona discharge treatment, plasma treatment and/or ultraviolet irradiation treatment is used.

The substrate sheet 10, 30 made from the metal, ceramics, or glass and the rubber sheet 20 are strongly bonded through the ether bond produced by the dehydration of the active groups, e.g. between the hydroxy groups, which are generated by the active treatment thereof. When the active groups such as the hydroxy groups are preliminarily exposed enough to form the ether bond by only stacking these sheets, the active treatment is not required.

Although the direct bond between the substrate sheet 10, 30 and the rubber sheet 20 through the ether bond is shown as one embodiment, the substrate sheet 10, 30 and the rubber sheet 20 may be indirectly bonded by the chemical bond such as the covalent bond or hydrogen bond through the silane-coupling agent. In this case, a single molecule of the silane-coupling agent can form the chemical bond by mediating between the substrate sheets 10, 30 and the rubber sheet 20. For example, at least any one of the bonded faces between the rubber sheet 20 made from the silicone rubber or non-silicone rubber and the substrate sheets 10, 30 made from the metal, ceramics, glass, or resin are activated by the corona discharge treatment, plasma treatment and/or ultraviolet irradiation treatment, and the substrate sheets 10, 30 and the rubber sheet 20 are bonded through the chemical bond which is mediated by the silane-coupling agent having an amino group and/or an alkoxy group having 1 to 4 carbons or an alkoxy equivalent group having hydrolyzability which may produce the ether bond by a reaction with the hydroxy group as well as these groups.

Thus, as a silane-coupling agent having an alkoxy group without an amino group, an available silane-coupling agent is included. Particularly, a silane-coupling agent having a vinyl group and alkoxy group exemplified by vinylmethoxysilane (KBM-1003) and vinyltriethoxysilane (KBE-1003); a silane-coupling agent having an epoxy group and alkoxy group exemplified by 2-(3,4-epoxycyclohexyl)ethyltrimethoxysilane (KBM-303), 3-glycidoxypropyl methyldimethoxysilane (KBM-402), 3-glycidoxypropyl trimethoxysilane (KBM-403), 3-glycidoxypropyl methyldiethoxysilane (KBE-402), and 3-glycidoxypropyl triethoxysilane (KBE-403); a silane-coupling agent having a styryl group and alkoxy group exemplified by p-styryltrimethoxysilane (KBM-1403); a silane-coupling agent having a (meth)acryl group and alkoxy group exemplified by 3-methacryloxypropyl methyldimethoxysilane (KBM-502), 3-methacryloxypropyl methyldiethoxysilane (KBM-503), 3-methacryloxypropyl methyldiethoxysilane (KBE-502), 3-methacryloxypropyl triethoxysilane (KBE-503), 3-acryloxypropyl trimethoxysilane (KBM-5103); a silane-coupling agent having an ureido group and alkoxy group exemplified by 3-ureidopropyltriethoxysilane (KBE-585); a silane-coupling agent having a mercapto group and alkoxy group exemplified by 3-mercaptopropylmethyldimethoxysilane (KBM-802) and 3-ercaptopropyltrimethoxysilane (KBM-803); a silane-coupling agent having a sulfide group and alkoxy group exemplified by bis(triethoxysilylpropyl) tetrasulfide (KBE-846); and a silane-coupling agent having an isocyanate group and alkoxy group exemplified by 3-isocyanatepropyltriethoxysilane (KBE-9007) (all of which is manufactured by Shin-Etsu Chemical Co., Ltd.; trade names) can be exemplified. Further, a silane-coupling agent having a vinyl group and acetoxy group exemplified by vinyltriacetoxysilane (Z-6075); a silane-coupling agent having an allyl group and alkoxy group exemplified by allyltrimethoxysilane (Z-6285); a silane-coupling agent having an alkyl group and alkoxy group exemplified by methyltrimethoxysilane (Z-6366), dimethyldimethoxysilane (Z-6329), trimethylmethoxysilane (Z-6013), methyltriethoxysilane (Z-6383), methyltriphenoxysilane (Z-6721), ethyltrimethoxysilane (Z-6321), n-propyltrimethoxysilane (Z-6265), diisopropyldimethoxysilane (Z-6258), isobutyltrimethoxysilane (Z-2306), diisobutyldimethoxysilane (Z-6275), isobutyltriethoxysilane (Z-6403), n-hexyltrimethoxysilane (Z-6583), n-hexyltriethoxysilane (Z-6586), cyclohexylmethyldimethoxysilane (Z-6187), n-octyltriethoxysilane (Z-6341), and n-decyltrimethoxysilane (Z-6210); a silane-coupling agent having an aryl group and alkoxy group exemplified by phenyltrimethoxysilane (Z-6124); a silane-coupling agent having an alkyl group and chlorosilane group exemplified by n-octyldimethylchlorosilane (ACS-8); a silane-coupling agent of an alkoxysilane exemplified by tetraethoxysilane (Z-6697) (all of which is manufactured by Dow Corning Toray Co., Ltd.; trade names) can be exemplified.

As a silane-coupling agent having an alkoxy group without an amino group, an alkoxysilyl compound having a hydrosilyl group (a SiH group) may be exemplified. For example,
(CH₃O)₃SiCH₂CH₂CH₂Si(CH₃)₂OSi(CH₃)₂H,
(C₂H₅O)₃SiCH₂CH₂CH₂Si(CH₃)₂OSi(CH₃)₂H,
(CH₃O)₃SiCH₂CH₂CH₂Si(OCH₃)₂OSi(OCH₃)₃,
(C₂H₅O)₃SiCH₂CH₂CH₂Si(OCH₃)₂OSi(OCH₃)₃,
(C₂H₅O)₃SiCH₂CH₂CH₂Si(CH₃)₂H,
(CH₃O)₃SiCH₂CH₂CH₂Si(CH₃)₂H,
(i-C₃H₇O)₃SiCH₂CH₂CH₂Si(CH₃)H₂,
(n-C₃H₇O)₃SiCH₂CH₂CH₂Si(CH₃)₂OSi(CH₃)₂CH₂CH₂Si(CH₃)₂Si(CH₃)₂H,
(n-C₄H₉O)₃SiCH₂CH₂CH₂Si(CH₃)₂OSi(CH₃)₂H,
(t-C₄H₉O)₃SiCH₂CH₂CH₂Si(CH₃)₂OSi(CH₃)₂H,
(C₂H₅O)₂CH₃SiCH₂CH₂CH₂Si(CH₃)₂OSi(CH₃)₂H,
(CH₃O)₂CH₃SiCH₂CH₂CH₂Si(CH₃)₂OSi(CH₃)₂CH₂CH₂Si(CH₃)₂Si(CH₃)₂H,
CH₃O(CH₃)₂SiCH₂CH₂CH₂Si(CH₃)₂OSi(CH₃)₂H,
(C₂H₅O)₃SiCH₂CH₂CH₂Si(CH₃)₂OSi(CH₃)₂H,
(n-C₃H₇)₃SiCH₂CH₂CH₂Si(CH₃)₂OSi(CH₃)₂H,
(i-C₃H₇O)₃SiCH₂CH₂CH₂Si(CH₃)₂OSi(CH₃)₂H,
(n-C₄H₉)₃SiCH₂CH₂CH₂Si(CH₃)₂OSi(CH₃)₂H,
(t-C₄H₉O)₃SiCH₂CH₂CH₂Si(CH₃)₂OSi(CH₃)₂H,
(C₂H₅O)₃SiCH₂CH₂Si(CH₃)₂OSi(CH₃)₂H,
(C₂H₅O)₃SiCH₂CH₂CH₂CH₂Si(CH₃)₂OSi(CH₃)₂H,
(C₂H₅O)₃SiCH₂CH₂CH₂CH₂CH₂CH₂Si(CH₃)₂OSi(CH₃)₂H,
(C₂H₅O)₃SiCH₂CH₂CH₂CH₂CH₂CH₂CH₂CH₂CH₂CH₂Si(CH₃)₂OSi(CH₃)₂H,
(CH₃O)₃SiCH₂C₆H₄CH₂CH₂Si(CH₃)₂C₆H₄Si(CH₃)₂H,
(CH₃O)₂CH₃SiCH₂C₆H₄CH₂CH₂Si(CH₃)₂C₆H₄Si(CH₃)₂H,
CH₃O(CH₃)₂SiCH₂C₆H₄CH₂CH₂Si(CH₃)₂C₆H₄Si(CH₃)₂H,
(C₂H₅O)₃SiCH₂C₆H₄CH₂CH₂Si(CH₃)₂C₆H₄Si(CH₃)₂H,
(C₂H₅O)₃SiCH₂CH₂CH₂Si(CH₃)₂C₆H₄OC₆H₄Si(CH₃)₂H,
(C₂H₅O)₃SiCH₂CH₂CH₂Si(CH₃)₂C₂H₄Si(CH₃)₂H,
(C₂H₅O)₃SiCH₂CH₂CH₂Si(CH₃)₂O[Si(CH₃)₂O]ₚ₁Si(CH₃)₂H,
C₂H₅O(CH₃)₂SiCH₂CH₂CH₂Si(CH₃)₂O[Si(CH₃)₂O]ₚ₂Si(C₂H₅)₂H,
(C₂H₅O)₂CH₃SiCH₂CH₂CH₂Si(CH₃)₂O[Si(CH₃)₂O]ₚ₃Si(CH₃)₂H,
(CH₃)₃SiOSiH(CH₃)O[SiH(CH₃)O]ₚ₄Si(CH₃)₃,
(CH₃)₃SiO[(C₂H₅OSi(CH₃)CH₂CH₂CH₂)SiCH₃]O[SiH(CH₃)O]ₚ₅Si(CH₃)₃,
(CH₃)₃SiO[(C₂H₅OSiOCH₃CH₂CH₂CH₂)SiCH₃]O[SiH(CH₃)O]ₚ₆Si(CH₃)₃,
(CH₃)₃SiO[(C₂H₅OSi(CH₃)CH₂CH₂CH₂)SiCH₃]O[SiH(CH₃)O]ₚ₇Si(CH₃)₃,
(CH₃)₃SiO[(Si(OC₂H₅)₂CH₂CH₂CH₂)SiCH₃]O[SiH(CH₃)O]ₚ₈Si(CH₃)₃,
(CH₃)₃SiOSi(OC₂H₅)₂O[SiH(CH₃)O]ₚ₉[Si(CH₃)₂O]_{q1}Si(CH₃)₃,
(CH₃)₃SiO[(C₂H₅Osi(CH₃)CH₂CH₂CH₂CH₂CH₂CH₂)Si(CH₃)O][SiH(CH₃)O]ₚ₁₀[Si(CH ₃)₂O]_{q2}Si(CH₃)₃,
(CH₃)₃SiO[(Si(OCH₃)₃CH₂CH₂CH₂CH₂CH₂CH₂)Si(CH₃)O][SiH(CH₃)O]ₚ₁₁[Si(CH₃)₂ O]_{q3}Si(CH₃)₃,
(CH₃)₃SiOSi(OC₂H₅)₂O[SiH(C₂H₅)O]ₚ₁₂Si(CH₃)₃,
(CH₃)₃SiO[(Si(OC₂H₅)₂CH₂CH₂CH₂CH₂CH₂CH₂)Si(C₂H₅)]O[SiH(C₂H₅)O]ₚ₁₃Si(CH₃) ₃,
(CH₃)₃SiO[(C₂H₅OSi(CH₃)CH₂CH₂CH₂CH₂CH₂CH₂)Si(C₂H₅)]O[SiH(C₂H₅)O]ₚ₁₄Si(C H₃)₃,
C₂H₅OSi(CH₃)₂CH₂CH₂CH₂CH₂CH₂CH₂(CH₃)₂SiO[HSi(CH₃)₂OSiC₆H₅O]ₚ₁₅Si(CH₃)₂ H,
Si(OCH₃)₃CH₂CH₂CH₂CH₂CH₂CH₂(CH₃)₂SiO[HSi(CH₃)₂OSiC₆H₅O]ₚ₁₆Si(CH₃)₂H,
H(CH₃)₂SiO[(C₂H₅OSi(CH₃)₂CH₂CH₂CH₂)Si(CH₃)O][HSiCH₃O]ₚ₁₇Si(CH₃)₂H,
H(CH₃)₂SiO[(C₂H₅OSi(CH₃)₂CH₂CH₂CH₂CH₂)Si(CH₃)O][HSiCH₃O]ₚ₁₈Si(CH₃)₂H,
H(CH₃)₂SiO[(C₂H₅OSi(CH₃)₂CH₂CH₂CH₂CH₂CH₂CH₂)Si(CH₃)O][HSiCH₃O]ₚ₁₉Si(C H₃)₂H,
H(CH₃)₂SiO[(C₂H₅OSi(CH₃)₂CH₂CH₂CH₂CH₂CH₂CH₂CH₂CH₂)Si(CH₃)O][HSiCH₃O ]ₚ₂₀Si(CH₃)₂H,
H(CH₃)₂SiO[(C₂H₅OSi(CH₃)₂CH₂CH₂CH₂CH₂CH₂CH₂CH₂CH₂CH₂CH₂)Si(CH₃)O][H SiCH₃O]ₚ₂₁Si(CH₃)₂H,
H(CH₃)₂SiO[(Si(OCH₃)₃CH₂CH₂C₆H₄CH₂CH₂)Si(CH₃)O][HSiCH₃O]ₚ₂₂Si(CH₃)₂H,
H(CH₃)₂SiO[(Si(OCH₃)₃CH₂C₆H₄CH₂CH₂CH₂)Si(CH₃)O][HSiCH₃O]ₚ₂₃Si(CH₃)₂H,
H(CH₃)₂SiO[(Si(OCH₃)₃CH₂C₆H₄CH₂CH₂)Si(CH₃)O][HSiCH₃O]ₚ₂₄Si(CH₃)₂H,
H(CH₃)₂SiO[(Si(OCH₃)₃C₆H₄CH₂CH₂)Si(CH₃)O][HSiCH₃O]ₚ₂₅Si(CH₃)₂H,
H(CH₃)₂SiO[(Si(OCH₃)₃CH₂CH₂CH₂)Si(CH₃)O][HSiCH₃O]ₚ₂₆Si(CH₃)₂H,
H(CH₃)₂SiO[(Si(OCH₃)₃CH₂CH₂CH₂CH₂)Si(CH₃)O][HSiCH₃O]ₚ₂₇Si(CH₃)₂H,
H(CH₃)₂SiO[(Si(OCH₃)₃CH₂CH₂CH₂CH₂CH₂CH₂)Si(CH₃)O][HSiCH₃O]ₚ₂₈Si(CH₃)₂H ,
H(CH₃)₂SiO[(Si(OCH₃)₃CH₂CH₂CH₂CH₂CH₂CH₂CH₂CH₂)Si(CH₃)O][HSiCH₃O]ₚ₂₉Si (CH₃)₂H,
H(CH₃)₂SiO[(Si(OCH₃)₃CH₂CH₂CH₂CH₂CH₂CH₂CH₂CH₂CH₂CH₂)Si(CH₃)O][HSiC H₃O]ₚ₃₀Si(CH₃)₂H,
H(CH₃)₂SiO[(Si(OCH₃)₃CH₂CH₂C₆H₄CH₂CH₂)Si(CH₃)O][HSiCH₃O]ₚ₃Si(CH₃)₂H,
H(CH₃)₂SiO[(Si(OCH₃)₃CH₂C₆H₄CH₂CH₂CH₂)Si(CH₃)O][HSiCH₃O]ₚ₃₂Si(CH₃)₂H,
H(CH₃)₂SiO[(Si(OCH₃)₃CH₂C₆H₄CH₂CH₂)Si(CH₃)O][HSiCH₃O]ₚ₃₃Si(CH₃)₂H,
H(CH₃)₂SiO[(Si(OCH₃)₃C₆H₄CH₂CH₂)Si(CH₃)O][HSiCH₃O]ₚ₃₄Si(CH₃)₂H,
H(CH₃)₂SiO[(Si(OCH₃)₃CH₂CH₂C₆H₄CH₂CH₂)Si(CH₃)O][HSiCH₃O]ₚ₃₅Si(CH₃)₂H,
H(CH₃)₂SiO[(CH₃O)Si(CH₃)CH₂CH₂CH₂CH₂CH₂CH₂Si(CH₃)₂OSiC₆H₅O]ₚ₃₆[HSi(CH ₃)₂OSiC₆H₅O]_{q4}Si(CH₃)₂H,
H(CH₃)₂SiO[Si(OCH₃)₂CH₂CH₂CH₂CH₂CH₂CH₂Si(CH₃)₂OSiC₆H₅O]ₚ₃₇[HSi(CH₃)₂O SiC₆H₅O]_{q5}Si(CH₃)₂H,
C₂H₅O(CH₃)₂SiO[SiH(CH₃)O]ₚ₃₈[SiCH₃(C₆H₅)O]_{q6}Si(CH₃)₂H,
Si(OC₂Hₛ)₃CH₂CH₂CH₂CH₂CH₂CH₂(CH₃)₂SiO[SiH(CH₃)0]p_{39[}SiCH₃(C₆Hₛ)0]q₇Si(C H₃)₂H,
C₂H₅OSi(CH₃)₂CH₂CH₂CH₂CH₂CH₂CH₂(CH₃)₂SiO[SiH(CH₃)O]ₚ₄₀[SiCH₃(C₆H₅)O]_{q8} Si(CH₃)₂H,
H(CH₃)₂SiO(C₂H₅O)Si(CH₃)O[SiH(CH₃)O]ₚ₄₁[SiCH₃(C₆H₅)O]_{q9}Si(CH₃)₂H, and
H(CH₃)₂SiO[Si(OC₂H₅)₃CH₂CH₂CH₂Si(CH₃)]O[SiH(CH₃)O]ₚ₄₂[SiCH₃(C₆H₅)O]_{q10}Si( CH₃)₂H
are optionally used. In these groups, p1 to p42 and q1 to q10 are number of 1 to 100. The alkoxysilyl compound having the hydrosilyl group preferably has the hydrosilyl group of 1 to 99 in a monomolecular thereof.

As a silane-coupling agent having alkoxy group without an amino group, an alkoxysilyl compound having hydrosilyl group can be exemplified. For example,
(C₂H₅O)₃SiCH₂CH=CH₂,
(CH₃O)₃SiCH₂CH₂CH=CH₂,
(C₂H₃O)₃SiCH₂CH₂CH=CH₂,
(CH₃O)₃SiCH₂CH₂CH₂CH₂CH=CH₂,
(C₂H₅O)₃SiCH₂CH₂CH₂CH₂CH=CH₂,
(C₂H₅O)₃SiCH₂CH₂CH₂CH₂CH₂CH₂CH=CH₂,
(CH₃O)₃SiCH₂(CH₂)₇CH=CH₂,
(C₂H₅O)₂Si(CH=CH₂)OSi(OC₂H₅)CH=CH₂,
(CH₃O)₃SiCH₂CH₂C₆H₄CH=CH₂,
(CH₃O)₂Si(CH=CH₂)O[SiOCH₃(CH=CH₂)O]ₜ₁Si(OCH₃)₂CH=CH₂,
(C₂H₅O)₂Si(CH=CH₂)O[SiOC₂H₅(CH=CH₂)O]ₜ₂Si(OC₂H₅)₃,
(C₂H₅O)₃SiCH₂CH₂CH₂Si(CH₃)₂OSi(CH₃)₂CH₂CH₂[Si(CH₃)₂O]ₜ₃CH=CH₂,
(CH₃O)₃SiCH₂CH₂CH₂Si(CH₃)₂OSi(CH₃)₂CH₂CH₂[Si(CH₃)₂O]ₜ₄CH=CH₂,
CH₃O(CH₃)₂SiCH₂CH₂CH₂Si(CH₃)₂OSi(CH₃)₂CH₂CH₂[Si(CH₃)₂O]ₜ₅CH=CH₂,
(C₂H₅O)₂CH₃SiCH₂CH₂CH₂Si(CH₃)₂OSi(CH₃)₂CH₂CH₂[Si(CH₃)₂O]ₜ₆CH=CH,
(C₂H₅O)₃SiCH₂CH₂CH₂Si(CH₃)₂OSi(CH₃)₂CH₂CH₂[Si(CH₃)₂O]ₜ₇CH=CH,
(C₂H₅O)₃SiCH₂CH₂CH₂Si(CH₃)₂OSi(CH₃)₂CH₂CH₂(Si(CH₃)₃O)Si(CH₃)O[SiCH₃(-)O] ᵤ₁Si(CH₃)₃CH=CH₂,
(C₂H₅O)₃SiCH₂CH₂CH₂Si(CH₃)₂OSi(CH₃)₂CH₂CH₂(Si(CH₃)₃O)Si(CH₃)O[SiCH₃(-)O] ᵤ₂[Si(CH₃)₂O]ₜ₈Si(CH₃)₃CH=CH₂,
(C₂H₅O)₂Si(CH=CH₂)O[SiCH₃(OC₂H₅)O]ᵤ₃Si(OC₂H₅)₂CH=CH₂,
(C₂H₅O)₂Si(CH=CH₂)O[Si(OC₂H₅)₂O]ᵤ₄Si(OC₂H₅)₂CH=CH₂, and
(C₂H₅O)₂Si(CH=CH₂)O[Si(OC₂H₅)₂O]ᵤ₅Si(OC₂H₅)₂CH=CH₂
are optionally used. In these groups, t1 to t8 and u1 to u5 are number of 1 to 30. The alkoxysilyl compound having the hydrosilyl group has preferably the vinyl group of 1 to 30 in the monomolecular thereof.

The reaction of these vinyl groups and SiH groups may be accelerated by the metal catalyst, e.g. a compound including platinum and thus, the substrate sheets and rubber sheet may be bonded.

As a silane-coupling agent having an alkoxy group without an amino group, an alkoxysilyl compound having an alkoxysilyl group at both terminals may be exemplified. For example,
(C₂H₅O)₃SiCH₂CH₂Si(OC₂H₅)₃,
(C₂H₅O)₂CH₃SiCH₂CH₂Si(OC₂H₅)₃,
(C₂H₅O)₃SiCH=CHSi(OC₂H₅)₃,
(CH₃O)₃SiCH₂CH₂Si(OCH₃)₃(CH₃O)₃SiCH₂CH₂C₆H₄CH₂CH₂Si(OCH₃)₃, (CH₃O)₃Si[CH₂CH₂]₃Si(OCH₃)₃,
(CH₃O)₂Si[CH₂CH₂]₄Si(OCH₃)₃,
(C₂H₅O)₂Si(OC₂H₅)₂,
(CH₃O)₂CH₃SiCH₂CH₂Si(OCH₃)₂CH₃,
(C₂H₅O)₂CH₃SiOSi(OC₂H₅)₂CH₃,
(CH₃O)₃SiO[Si(OCH₃)₂O]ᵥ₁Si(OCH₃)₃,
(C₂H₅O)₃SiO[Si(OC₂H₅)₂O]ᵥ₂Si(OC₂H₅)₃, and
(C₃H₇O)₃SiO[Si(OC₃H₇)₂O]ᵥ₃Si(OC₃H₇)₃
are optionally used. In these groups, v1 to v3 are number of 0 to 30.

As a silane-coupling agent having an alkoxy group without an amino group, an alkoxysilyl compound having hydrolytic group-containing silyl group can be exemplified. For example, an easily-hydrolytic organosilane is optionally used. Particularly,
CH₃Si(OCOCH₃)₃, (CH₃)₂Si(OCOCH₃)₂, n-C₃H₇Si(OCOCH₃)₃, CH₂=CHCH₂Si(OCOCH₃)₃, C₆H₅Si(OCOCH₃)₃, CF₃CF₂CH₂CH₂Si(OCOCH₃)₃, CH₂=CHCH₂Si(OCOCH₃)₃, CH₃OSi(OCOCH₃)₃, C₂H₅OSi(OCOCH₃)₃, CH₃Si(OCOC₃H₇)₃, CH₃Si[OC(CH₃)=CH₂]₃, (CH₃)₂Si[OC(CH₃)=CH₂]₃, n-C₃H₇Si[OC(CH₃)=CH₂]₃, CH₂=CHCH₂Si[OC(CH₃)=CH₂]₃, C₆H₅Si[OC(CH₃)=CH₂]₃, CF₃CF₂CH₂CH₂Si[OC(CH₃)=CH₂]₃, CH₂=CHCH₂Si[OC(CH₃)=CH₂]₃, CH₃OSi[OC(CH₃)=CH₂]₃, C₂H₅OSi[OC(CH₃)=CH₂]₃, CH₃Si[ON=C(CH₃)C₂H₅]₃, (CH₃)₂Si[ON=C(CH₃)C₂H₅]₂, n-C₃H₇Si[ON=C(CH₃)C₂H₅]₃, CH₂=CHCH₂Si[ON=C(CH₃)C₂H₅]₃, C₆H₅Si[ON=C(CH₃)C₂H₅]₃, CF₃CF₂CH₂CH₂Si[ON=C(CH₃)C₂H₅]₃, CH₂=CHCH₂Si[ON=C(CH₃)C₂H₅]₃, CH₃OSi[ON=C(CH₃)C₂H₅]₃, C₂H₅OSi[ON=C(CH₃)C₂H₅]]₃, CH₃Si[ON=C(CH₃)C₂H₅]₃, CH₃Si[N(CH₃)]₃, (CH₃)₂Si[N(CH₃)]₂, n-C₃H₇Si[N(CH₃)]₃, CH₂=CHCH₂Si[N(CH₃)]₃, C₆H₅Si[N(CH₃)]₃, CF₃CF₂CH₂CH₂Si[N(CH₃)]₃, CH₂=CHCH₂Si[N(CH₃)]₃, CH₃OSi[N(CH₃)]₃, C₂H₅OSi[N(CH₃)]₃, and CH₃Si[N(CH₃)]₃ are included.

As a silane-coupling agent containing an amino group and having alkoxy group, an available silane-coupling agent is included. Particularly, an alkoxysilyl compound containing the amino group exemplified by N-2-(aminoethyl)-3-aminopropylmethyldimethoxysilane (KBM-602), N-2-(aminoethyl)-3-aminopropyltrimethoxysilane (KBM-603), N-2-(aminoethyl)-3-aminopropyltriethoxysilane (KBE-603), 3-aminopropyltrimethoxysilane (KBM-903), 3-aminopropyltriethoxysilane (KBE-903), 3-triethoxysilyl-N-(1,3-dimethyl-butylidene) propylamine (KBE-9103), N-phenyl-3-aminopropyltrimethoxysilane (KBM-573), and N-(vinylbenzyl)-2-aminoethyl-3-aminopropyltrimethoxysilane hydrochloride (KBM-575) (all of which is manufactured by Shin-Etsu Chemical Co., Ltd.; trade names) may be used. Further, an alkoxysilyl compound containing an amino group exemplified by 3-aminopropyltrimethoxysilane (Z-6610), 3-aminopropyltrimethoxysilane (Z-6611), 3-(2-aminoethyl)aminopropyltrimethoxysilane (Z-6094), 3-phenylaminopropyltrimethoxysilane (Z-6883), and N[3-(trimethoxysilyl)propyl]-N'-[(ethenylphenyl)methyl]-1,2-ethanediamine hydrochloride (Z-6032) (all of which is manufactured by Dow Corning Toray Co., Ltd.; trade names) may be used.

When the substrate sheets 10, 30 are made from the metal, ceramics, or glass, and the rubber sheet 20 is made from the silicone rubber, these sheets are preferably bonded through the direct ether bond. In this case, the active groups such as the hydroxy group are generated on the faces of the substrate sheets 10, 30 and the rubber sheet 20 by the corona discharge treatment and thus, the ether bond is formed between the substrate sheets 10, 30 and the rubber sheet 20 through the dehydration thereof by compression bond under pressurization or reduced pressure.

When the substrate sheets 10, 30 are made from the metal, ceramics, or glass, and the rubber sheet 20 is made from the non-silicone rubber, these sheets are preferably bonded by a covalent bond of an oxygen-carbon bond, carbon-carbon bond, and oxygen-silicon bond through the silane-coupling agent having the alkoxy group without the amino group. In this case, the active groups such as the hydroxy group are generated on the faces of the substrate sheets 10, 30 and the rubber sheet 20 by the corona discharge treatment and thus, the covalent bonds are formed by the compression bond under normal atmospheric pressure, the pressurization, or reduced pressure at normal temperature or heating temperature by applying the silane-coupling agent including the alkoxy group or the alkoxy-equivalent group, and optionally a unsaturated group, epoxy group, ureido group, sulfide group, or isocyanate group without the amino group.

When the substrate sheets 10, 30 are made from the resin, and the rubber sheet 20 is made from the silicone rubber or non-silicone rubber, these sheets are preferably bonded by chemical bonds of the covalent bond of the oxygen-silicon bond through the silane-coupling agent having the amino group and the alkoxy group, and the hydrogen bond of the hydroxy group-amino group; and a covalent bond such as an amide bond or imino bond by a carboxyl group or carbonyl group which is newly produced. In this case, the active groups such as the hydroxy group are generated on the faces of the substrate sheets 10, 30 and the rubber sheet 20 by conducting the corona discharge treatment; the silane-coupling agent including the alkoxy group or an alkoxy-equivalent group and the amino group is applied to these sheets; and when these sheets are compressed under the normal atmospheric pressure, pressurization, or reduced pressure at the normal temperature or heating temperature, these chemical bonds are produced. In this instance, the amino group of the silane-coupling agent is easily adsorbed to the resin. When the resin is the polycarbonate resin, the cycloolefin resin, the polyethylene terephthalate resin, the acryl resin, or the epoxy resin, these sheets are rapidly, strongly and easily bonded by being especially progressed the reaction. Among them, when the resin is the polycarbonate resin or the polycarbonate resin, superior water resistance is exhibited.

Approach of the active groups such as the hydroxy group of the substrate sheets 10, 30 and the hydroxy group of the rubber sheet 20 or a reactive functional group of the silane-coupling agent, which reacts therewith, is accelerated by removing gaseous media of contact boundaries under reduced pressure or vacuum conditions, for example, 50torr or less, more particularly, the reduced pressure conditions of 50 to 10torr, or the vacuum conditions of less than 10torr, more particularly, less than 10torr to 1x10⁻³torr, preferably less than 10torr to 1x10⁻²torr, or by adding a stress (a load) e.g. 10 to 200kgf to the contact boundaries thereof, and further by heating the contact boundaries thereof. The whole surfaces of the bonded faces of the hydroxy group of the substrate sheet 10, 30 and the rubber sheet 20 are preferably homogeneously pressurized. If the values fall outside the above range, the pressure could not homogeneously be applied.

Thus, the microchemical chip 1 is produced as follows, as shown fig.1 which shows one embodiment thereof.

The silicone rubber sheet 20 is cut so as to shape a rectangular parallelepiped. The micro flow path 26 is formed to be penetrated into the rubber sheet 20 by boring trough the laser beam machining. By the laser beam machining, the flow path 26 is shaped so as to extend from the fluid sample injection parts 21a, 21b of the starting point terminals; to join at a downstream of these parts; to have the branch channel which extends therefrom to the fluid sample drain part 22a and the main channel which extends therefrom to the fluid sample drain parts 22b, 22c; to divide at a downstream of the main channel; and to extend at the downstream to the fluid sample drain parts 22b, 22c of the ending point terminals. Next, the metal substrate sheet 10 for covering is cut so as to have the same size as the rubber sheet 20. The fluid sample injection holes 11a, 11b and the fluid sample drain holes 12a, 12b, 12c are opened in position corresponding to the fluid sample injection parts 21a, 21b and the fluid sample drain part 22a, 22b, 22c of the metal substrate sheet 10 by drilling or punching, respectively. Further next, the metal substrate sheet 30 for supporting the bottom face is cut so as to have the same size as the rubber sheet 20.

The substrate sheet 10, 30 and rubber sheet 20 are washed with alcohol or water. When the surfaces of the lower face 15 of the substrate sheet 10, the upper face 34 of the substrate sheet 30, and the both faces 24, 25 of the rubber sheet 20 are treated by the corona discharge treatment, the hydroxy groups are newly generated thereto. The rubber sheet 20 is sandwiched between the substrate sheets 10, 30, and the pressure is reduced e.g. at 10torr or less. Next, these sheets are thermally compressed and bonded by heating e.g. at 80 to 120°C while pressing e.g. at 10 to 200kgf. In the result, the ether bond is generated by the dehydration between the hydroxy groups of the substrate sheet 10, 30 and the hydroxy groups of the rubber sheet 20 and these sheets are bonded. Thus, the microchemical chip 1 is obtained.

Incidentally, although the embodiment, which is conducted with the corona discharge treatment to the substrate sheets 10, 30 and the rubber sheet 20, is shown, it may be conducted with the atmospheric pressure plasma treatment and/or the ultraviolet irradiation treatment. The active group of the hydroxy group is produced on the surfaces of the organic or inorganic substrate sheets 10, 30 and the rubber sheet 20 by these treatments. Further, the active group exemplified by carboxyl group or carbonyl group is produced on the surfaces of the organic substrate sheets 10, 30 and the rubber sheet 20 thereby.

The substrate sheets 10, 30 and the rubber sheet 20 originally have the hydroxy group or do not originally have the hydroxy group. When these sheets do not have the hydroxy group on the surfaces thereof, the hydroxy group is effectively produced thereon by conducting the corona discharge treatment, atmospheric pressure plasma treatment, or ultraviolet irradiation treatment.

The optimal treatment conditions vary according to the history and kinds of the materials of the surfaces of the substrate sheets 10, 30 and the rubber sheet 20. It is important to conduct the treatment until obtaining a surface tension up to 55kJ/m continuously. A sufficient adhesive strength is obtained thereby.

Particularly, the corona discharge treatment of the substrate sheets 10, 30 and the rubber sheet 20 is conducted under conditions of e.g. power source: 100V, output voltage: 0 to 20kV, oscillating frequency: 0 to 40kHz for 0.1 to 60 seconds, and temperature: 0 to 60°C by using an apparatus for corona surface modification (e.g. CoronaMaster produced by Shinko Electric & Instrumentation Co., Ltd.).

The atmospheric pressure plasma treatment of the substrate sheets 10, 30 and the rubber sheet 20 is conducted under conditions of e.g. plasma processing speed: 10 to 100mm/s, power source: 200 or 220V AC (30A), compressed air: 0.5MPa (1NL/min.), and 10kHz/300W to 5GHz, electric power: 100 to 400W, and irradiation period of time: 1 to 60 seconds by using an air plasma generator (e.g. trade name of Aiplasma produced by Matsushita Electric Industrial Co., Ltd.).

The ultraviolet irradiation treatment of the substrate sheets 10, 30 and the rubber sheet 20 is conducted under conditions of e.g. wave length: 200 to 400nm, power source: 100V AC, light source peak illuminance: 400 to 3000mW/cm², irradiation period of time: 0.1 to 60 seconds by using an ultraviolet-light emitting diode (UV-LED) irradiator (e.g. UV irradiator: trade name of ZUV-C30H produced by OMRON Corporation).

After activation treatment such as the corona discharge treatment, the surfaces 15, 34 of the substrate sheets 10, 30, which should be bonded, are dipped into the silane-coupling agent of a molecular adhesive agent, the silane-coupling agent is sprayed onto the surfaces 15, 34, and the substrate sheets 10, 30 and the rubber sheet 20 may be contacted thereafter. The period of time of dipping or spraying is not restricted, it is important to homogeneously wet the substrate surfaces of the substrate sheets 10, 30.

The substrate sheets 10, 30, which are applied the silane-coupling agent, are dried while heating by putting in an oven, by blasting the air using a dryer, or by irradiating high frequency wave. The heating and drying are conducted at a temperature range of 50 to 250°C for 1 to 60 minutes. If the temperature is less than 50°C, the reaction between the hydroxy group generated on the surfaces of the substrate sheets 10, 30 and the silane-coupling agent takes a long time, decreasing in productivity, and increasing in cost. On the other hand, if the temperature is more than 250°C, the surfaces of the substrate sheets 10, 30 are deformed or degraded even for short period of time. If the time of heating and drying is less than 1 minute, thermal conductivity is insufficient and thus, the hydroxy group of the surfaces of the substrate sheets 10, 30 and the silane-coupling agent are insufficiently bonded. On the other hand, if the time thereof is more than 60 minute, the productivity decreases.

When the reaction between the hydroxy group of the surfaces of the substrate sheets 10, 30 and the silane-coupling agent is insufficient, the dipping and drying may be repeated about 1 to 5 times. Therefore, the time of the dipping and drying of each time can decrease and thus, the reaction can be sufficiently progressed by increasing the reaction frequency.

According to explanation with reference to Fig. 1, for example in the case of microsynthesis, the microchemical chip 1 is used as follows. The microchemical chip 1 is installed to an apparatus body of a microreactor (not shown) of a reaction device. A pressurizer is used to connect to holes for injecting the fluid sample, for flowing the fluid sample into the flow path by pressurization after injecting the fluid sample. Syringes (not shown) are air-tightly inserted in injection holes 11a, 11b of the substrate sheet 10 for covering. And the fluid samples of the liquid specimen and liquid reagent are imported from the syringes into the flow path 26 through the fluid sample injection parts 21a, 21b while these are separately pressurized at a pressure between more than 100kPa to 3MPa or less, respectively. Both fluid samples join by flowing in the flow path 26, mix, and mutually react. A waste fluid is optionally drained from the fluid sample drain hole 12a through the fluid sample drain part 22a of the branch channel. The fluid sample containing the infinitesimally-synthesized resultant product is drained and thus, an objective product is obtained.

A reaction device of the present invention is at least composed of the microchemical chip 1, an apparatus body for installing the microchemical chip 1, and a pressurizer for pressurizing the fluid sample after injecting it into the microchemical chip 1. The pressurizer has an injector such as a syringe which is connected to a hole for injecting the fluid sample, and a fluid machine such as a pump for sending the fluid sample. The fluid sample can be injected and flowed in the flow path 26 by using the pressurizer. It is preferable that a flow velocity is 0.1 to 500µl/min. The reaction device may have a heating mechanism such as a heater and/or a cooling mechanism which contact with the microchemical chip 1 or do not contact therewith at the upper side and the lower side thereof.

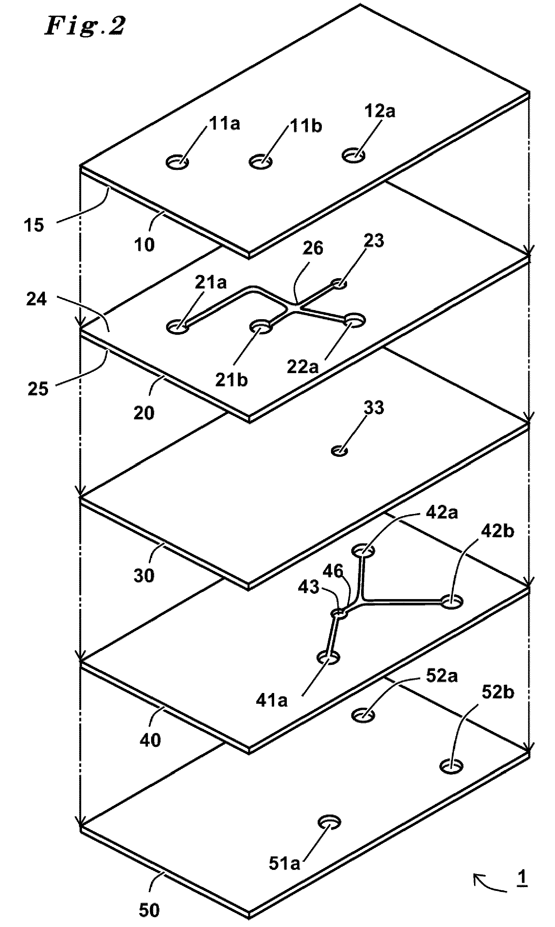

Another embodiment of a microchemical chip 1 is shown in Fig. 2. In the microchemical chip 1, a metal substrate sheet 10 for covering, a first rubber sheet 20, a substrate sheet 30 for a liner medium, a second rubber sheet 40, and a metal substrate sheet 50 for supporting the bottom face are stacked in this order.

Flow paths 26, 46 are formed into the rubber sheets 20, 40 by penetrating both faces thereof. In the rubber sheet 20, the flow path 26 is extended from fluid sample injection parts 21a, 21b of the starting point terminals, respectively; joined at a downstream of these parts; and divided into a branch channel extended therefrom to a fluid sample drain part 22a and a main channel extended therefrom to a fluid sample transfer part 23. A fluid sample transfer hole 33 is opened into the metal substrate sheet 30 for a liner medium in position to corresponding to the fluid sample transfer part 23. The fluid sample transfer hole 33 may have a check valve. A fluid sample import part 43 is formed into the second rubber sheet 40 in position corresponding to the fluid sample transfer hole 33, and a flow path 46 is formed thereinto by penetrating the both faces thereof. The flow path 46 is extended from a fluid sample injection part 41a of the other starting point terminal to the fluid sample import part 43; joined thereat; divided in a downstream thereof; and extended to fluid sample drain parts 42a, 42b of ending point terminals. A fluid sample injection hole 51a and fluid sample drain holes 51b, 51c are opened into the metal substrate sheet 50 for supporting the bottom face in position corresponding to the fluid sample injection part 41a and the fluid sample drain parts 42a, 42b. The substrate sheets 10, 20, 30 and the rubber sheets 20, 40 are directly bonded through the ether bond as well as Fig. 1. The substrate sheets 10, 20, 30 and the rubber sheets 20, 40 may be made from the above raw material, may have the above shape, and may be bonded through the silane-coupling agent. The microchemical chip 1 is used in the manner of importing the fluid sample by pressurization as well as Fig. 1. When the fluid samples are respectively different for each of molecular weight, composition of components, and physical properties thereof in injecting them, the microchemical chip 1 can prevent unexpected contamination by each of the flow paths 26, 46 of the plural rubber sheets 20, 40. Further, the microchemical chip 1 may appropriately separate the fluid samples corresponding to variety of molecular weight of objective substance and/or variety of specific weight thereof in the fluid sample by reaction at the flow paths 26, 46.

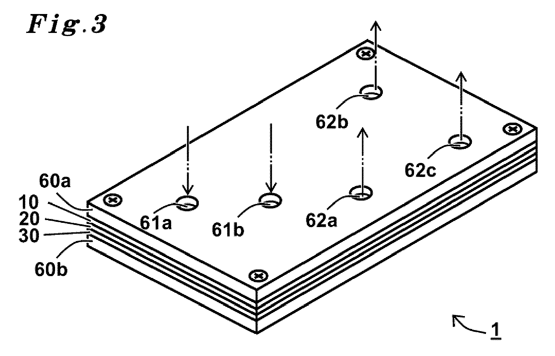

Another embodiment of a microchemical chip 1 is shown in Fig. 3. The microchemical chip 1 consists of the substrate sheets 10, 30 and the rubber sheet 20 shown in Fig. 1. The outermost substrate sheets 10, 30 with the rubber sheet 20 are sandwiched between holders 60a, 60b made of two resin plates or metal plates having rigidity and inflexibility. These plates are screwed to be fixed. Injection induction holes 61a, 61b and drain induction holes 62a, 62b, 62c are opened into the holders 60a, 60b in position corresponding to the fluid sample injection holes 11a, 11b and fluid sample drain holes 12a, 12b, 12c of the substrate sheets 10, 30, respectively. The microchemical chip 1 is used in the manner of importing the fluid sample to the flow path 26 by pressurization as well as Fig. 1. The holders 60a, 60b press the flexible substrate sheets 10, 30 and the rubber sheet 20 so as to be able to flow the fluid sample to the flow path 26 and correct these sheets so as to not curve. The microchemical chip 1 may have the substrate sheets 10, 30, 50 and the rubber sheets 20, 40 as shown in Fig. 2. In the microchemical chip 1 shown in Figs. 1 and 2, the heater (not shown) may be inserted and bonded between the substrate sheet 10, 30 and the rubber sheet 20, the heater may be arranged on the holders as shown in Fig. 3. A sensor such as an electrode etc. for detecting the specimen, the reagent, and/or the reaction product may be wired in any one of the fluid sample injection parts 21 a, 21b, the fluid sample drain parts 22a, 22b, 22c, the fluid sample injection part 41a, and the fluid sample drain parts 42a, 42b.

### Embodiments

Embodiments of the present invention will be described in detail below, but the scope of the present invention is not restricted to these embodiments.

### (Example 1)

A microchemical chip 1 shown in Fig. 1 was produced by employing cycloolefin rein substrate sheets 10, 30 and a silicone rubber sheet 20. The cycloolefin resin substrate sheets 10, 30 were formed by ZEONOR as a cycloolefin resin (registered trademark, produced by Zeon Corporation) and had 2mm in the thickness and 30 x 40mm in size. The silicone rubber sheet 20 was formed by SH-851-U as polydimethylsiloxane (trade name, produced by Dow Corning Toray Co., Ltd.) was shaped in the same shape as the cycloolefin resin substrate sheets 10, 30, and had 50µm in the thickness. Just like Fig. 1, a channel-shaped and branched flow path 26 having 500µm in width, which had fluid sample injection parts 21a, 21b and fluid sample drain parts 22a, 22b, 22c having 1mm in a diameter respectively, was formed by using a laser beam machining apparatus (model: LaserPro SPIRIT, produced by COMNET Corporation, process conditions: speed 10, power 30, and PPI 400). The fluid sample injection holes 11a, 11b and the fluid sample drain holes 12a, 12c were drilled into the substrate sheet 10 for covering. After the substrate sheet 10 for covering and the substrate sheet 30 for supporting bottom face were washed with ethanol and water, the surfaces of the substrate sheets 10, 30 were activated by a corona discharge treatment 3 times under conditions of 1mm in a gap length, 13.5kV in a voltage, and 70mm/sec. After the substrate sheets 10, 30 were dipped into an ethanol solution of 0.1% 3-(2-aminoethylamino)propyltrimethoxysilane of a silane-coupling agent by weight, the substrate sheets 10, 30 were washed with ion-exchanged water, dried by an air gun, heated at 80°C for 10 minutes, washed with ethanol again, treated with 3-(2-aminoethylamino)propyltrimethoxysilane and drying, and then treated by the corona discharge treatment under the same conditions. The rubber sheet 20 was sandwiched between the substrate sheets 10, 30 while the fluid sample injection holes 11a, 11b and the fluid sample drain holes 12a, 12b, 12c were positioned on the fluid sample injection parts 21a, 21b and the fluid sample drain parts 22a, 22b, 22c. After the resultant sheets were exposed under reduced pressure conditions of 10torr for 15 seconds, the resultant sheets were thermally compressed and bonded by pressing in 70kgf at 80°C for 15 seconds to obtain the microchemical chip 1.

When the fluid sample injection hole 11b and the fluid sample drain holes 12a, 12b, 12c were closed and then a compressed air was introduced from the fluid sample injection part 21a through the fluid sample injection hole 11a, a pressure resistance was exhibited up to 1.5MPa.

### (Example 2)

A microchemical chip 1 shown in Fig. 1 was produced by employing stainless sheets 10, 30 having 30mm in horizontal and vertical sides, and 2mm in the thickness, respectively, and the silicone rubber sheet 20 having the same shape therewith and 50µm in the thickness. A flow path 26 having fluid sample injection parts 21a, 21b, 21c and fluid sample drain parts 22a, 22b, 22c was formed by using the laser beam machining apparatus, just like Fig. 1. Fluid sample injection holes 11a, 11b and fluid sample drain holes 12a, 12b, 12c were drilled into the substrate sheet 10. The substrate sheets 10, 30 were washed with ethanol and water. After the substrate sheets 10, 30 and the rubber sheet 20 were washed with ethanol and water, the surfaces of these sheets were activated by the corona discharge treatment in the same conditions as Example 1. The rubber sheet 20 was sandwiched between the substrate sheets 10, 30 while the fluid sample injection holes 11a, 11b and the fluid sample drain holes 12a, 12b, 12c were positioned on the fluid sample injection parts 21a, 21b and the fluid sample drain parts 22a, 22b, 22c. After the resultant sheets were exposed under reduced pressure conditions of 10torr for 15 seconds, the resultant sheets were thermally compressed and bonded by pressing in 70kgf at 80°C for 15 seconds to obtain a microchemical chip 1 was obtained.

A pressure resistance was exhibited as well as the microchemical chip in Example 1.

### (Example 3)

### (1) Production of a microchemical chip

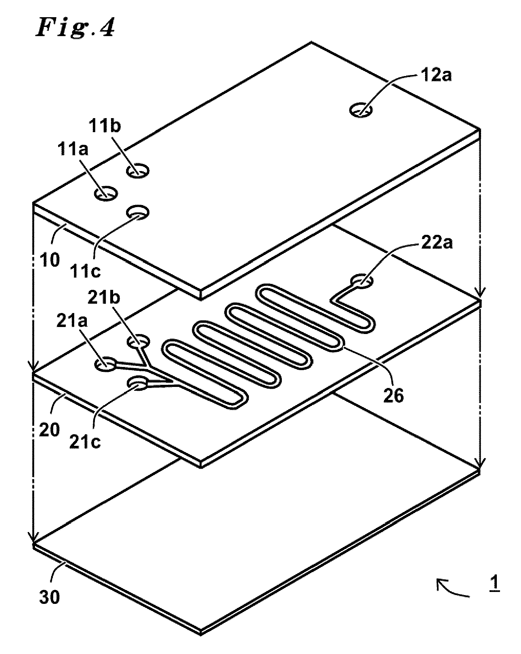

A microchemical chip 1 shown in Fig. 4 was produced by employing cycloolefin resin substrate sheets 10, 30 and the silicone rubber sheet 20. The cycloolefin resin substrate sheet 10, 30 were formed by ZEONOR as a cycloolefin resin (registered trademark, produced by Zeon Corporation). The cycloolefin resin substrate sheet 10 had 2mm in the thickness and the cycloolefin resin substrate sheet 30 had 188µm in the thickness, and the both substrate sheets had 30 x 40mm in size. The silicone rubber sheet 20 was formed by SH-851-U as polydimethylsiloxane (trade name, produced by Dow Corning Toray Co., Ltd.) was shaped in the same shape as the cycloolefin resin sheet substrate sheets 10, 30, and had 500µm in the thickness. Just like Fig. 4, a channel-shaped and branched flow path 26 having 500µm in width, which had the fluid sample injection parts 21a, 21b, 21c and the fluid sample drain part 22a having 1mm in a diameter, respectively, was formed by using a laser beam machining apparatus (model: LaserPro SPIRIT, produced by COMNET Corporation, process conditions: speed 10, power 30, and PPI 400). The fluid sample injection holes 11a, 11b, 11c and the fluid sample drain hole 12a were drilled into the substrate sheet 10 for covering. After the substrate sheet 10 for covering and the substrate sheet 30 for supporting bottom face were washed with ethanol and water, the surfaces of the substrate sheets 10, 30 were activated by the corona discharge treatment 3 times under conditions of a gap length of 1mm, a voltage of 13.5kV, and 70mm/sec. After the substrate sheets 10, 30 were dipped into an ethanol solution of 0.1% 3-(2-aminoethylamino)propyltrimethoxysilane (AEAPS) of a silane-coupling agent by weight, the resultant sheets were dried by the air gun, heated at 80°C for 10 minutes, washed with ethanol again, treated with 3-(2-aminoethylamino)propyltrimethoxysilane and drying, and then treated by the corona discharge treatment under the same conditions. The rubber sheet 20 was sandwiched between substrate sheets 10, 30 while the fluid sample injection holes 11a, 11b and the fluid sample drain holes 12a, 12b, 12c were positioned on the fluid sample injection parts 21a, 21b and the fluid sample drain parts 22a, 22b, 22c. After the resultant sheets were exposed under reduced pressure conditions of 10torr for 15 seconds, the resultant sheets were thermally compressed and bonded by pressing in 70kgf at 80°C for 15 seconds to obtain a microchemical chip.

### (2) Preparation of each reagent

Fluid sample A: 7g of copper sulfate (II) pentahydrate (produced by Wako Pure Chemical Industries, Ltd.) was dissolved in 100mL of ion-exchanged water.
Fluid sample B: 35g of potassium sodium tartrate (produced by Wako Pure Chemical Industries, Ltd.) and 10g of sodium hydrate (produced by Wako Pure Chemical Industries, Ltd.) were dissolved in 100mL of the ion-exchanged water.
Fluid sample C: 35.0 to 38.0% of a formaldehyde solution (produced by Wako Pure Chemical Industries, Ltd.).

### (3) Reaction in the microchemical chip

After the produced microchemical chip was preheated for 5 minutes on a metal plate which was heated at 90°C, the prepared fluid samples A, B, and C were introduced from the fluid sample injection holes 11a, 11b, 11c at 3 µl/min., 3µl/min., and 1µl/min. of flow velocity by using a pressurizer, respectively. The microchemical chip was left to stand for predetermined period of time. When waste fluid, which was drained from the fluid sample drain hole 12a, was visually observed, a discoloration to red-brown was indicated and thus, mixing and reacting of the liquid samples in the microchemical chip were confirmed.

### Industrial Applicability

The microchemical chip of the present invention can be used in an analysis of biological component of patients at an emergency medical practice which requires to obtain a result of the analysis rapidly; a DNA analysis for identification of DNA using a electrophoresis after extracting DNA from things left behind such as a trace amount of a bloodstain, biological fluid, hair, and a biological tissue cell etc. at a crime scene, and conducting a PCR amplification for amplifying DNA; an evaluation of physical properties and drug efficacy of various drug candidates for searching a novel drug; diagnosis for custom-made medical treatment; microsynthesis of peptide, DNA, and a functional low molecule, and the like.

The microchemical chip can be used in custom-made medical care, identification by a DNA analysis of various flora and fauna etc., because the flow path can be easily formed so as to have various shapes.

The obtained microchemical chip by the method for producing the microchemical chip of the present invention after installing it onto a microreactor or analysis apparatus can be used to conduct a genetic diagnosis or healing in a medical field; various analyses in a criminal investigation field by using biological reagent; microbiological search by using an underwater apparatus in a remote location such as the ocean or lake and a reservoir etc.; and various syntheses of drug development.

The reaction device of the present invention for conducting an analytic reaction or synthetic reaction of the trace amount of the specimen and/or the reagent, especially, can be used as the analysis apparatus or microreactor.

### Explanations of Letters or Numerals

1: microchemical chip, 10: substrate sheet, 11a, 11b: fluid sample injection hole, 12a, 12b, 12c: fluid sample drain hole, 15: lower face, 20: rubber sheet, 21a, 21b: fluid sample injection part, 22a, 22b, 22c: fluid sample drain part, 23: fluid sample transfer part, 24: upper face, 25: lower face, 26: flow path, 27: side surface, 30: substrate sheet, 33: fluid sample transfer hole, 34: upper face, 40: rubber sheet, 41a: fluid sample injection part, 42a, 42b: fluid sample drain part, 43: fluid sample import part, 50: substrate sheet, 51a: fluid sample injection hole, 52a, 52b: fluid sample drain hole, 60a, 60b: holder, 61a, 61b: injection induction hole, 62a, 62b, 62c: drain induction hole

## Claims

1. A microchemical chip comprising:
a rubber sheet having a penetrated flow path which chemically reacts a pressurized fluid sample selected from a specimen and a reagent by flowing thereinto;
substrate sheets which sandwich the rubber sheet and bond to both faces thereof by direct bond or by chemical bond through a silane-coupling agent and are selected from the group consisting of metal, ceramics, glass, and resin; and
a hole for injecting the fluid sample into the flow path and a hole for draining the fluid sample flowed therefrom which are opened into the substrate sheet.

2. The microchemical chip according to claim 1, wherein the rubber sheet and the substrate sheets are bonded by the chemical bond which is formed under conditions of reduced pressure and/or pressurization.

3. The microchemical chip according to claim 1 or 2, wherein the rubber sheet and the substrate sheets are bonded by the chemical bond which is formed under conditions of pressurization and/or heating after reduced pressure condition.

4. The microchemical chip according to at least one of claims 1 to 3, wherein the rubber sheet and/or the substrate sheet are given an active treatment at these bonded faces.

5. The microchemical chip according to at least one of claims 1 to 4, wherein a plurality of the rubber sheets which are sandwiched between the substrate sheets is composed by stacking thereof.

6. The microchemical chip according to at least one of claims 1 to 5, wherein the outermost substrate sheets are sandwiched between plate-shaped holders, and fixed with the rubber sheet so as to inhibit leakage of the fluid sample.

7. The microchemical chip according to at least one of claims 1 to 6, wherein the rubber sheet is made from silicone rubber.

8. The microchemical chip according to at least one of claims 1 to 7, wherein the rubber sheet which is made from silicone rubber and the substrate sheets are activated by a corona discharge treatment, a plasma treatment and/or an ultraviolet irradiation treatment over at least any one of these bonded faces, and bonded by the direct bond.

9. The microchemical chip according to at least one of claims 1 to 7, wherein the rubber sheet which is made from silicone rubber or non-silicone rubber and the substrate sheets are activated by a corona discharge treatment, a plasma treatment and/or an ultraviolet irradiation treatment over at least any one of these bonded faces, and bonded by the chemical bond through the silane-coupling agent having an amino group and/or an alkoxy group.

10. The microchemical chip according to claim 9, wherein the substrate sheets are made from the resin which is at least one selected from the group consisting of a polycarbonate resin, cycloolefin resin, polyethylene terephthalate resin, acryl resin, and epoxy resin;
the silane-coupling agent has the amino group and alkoxy group.

11. The microchemical chip according to at least one of claims 1 to 10, wherein at least a side surface of the flow path of the rubber sheet is coated by coating.

12. A method for producing a microchemical chip comprising the steps of:
a flow path forming step of forming a flow path, which reacts a pressurized fluid sample selected form a specimen and a reagent by flowing thereinto, into a rubber sheet by penetrating it;
a perforating step of forming a hole for injecting the fluid sample into the flow path and a hole for draining the fluid sample flowed therefrom into the substrate sheet selected from the group consisting of metal, ceramics, glass, and resin; and
a bonding step of bonding the substrate sheets to both faces of the rubber sheet by direct bond or by chemical bond through a silane-coupling agent while sandwiching the rubber sheet therebetween.

13. The method for producing the microchemical chip according to claim 12, wherein the rubber sheet is bonded to the substrate sheets by the chemical bond under reduced pressure.

14. A reaction device comprising:
a microchemical chip comprising a rubber sheet having a penetrated flow path which chemically reacts a pressurized fluid sample selected from a specimen and a reagent by flowing thereinto; substrate sheets which sandwich the rubber sheet, and bond to both faces thereof by direct bond or by chemical bond through a silane-coupling agent and are selected from the group consisting of metal, ceramics, glass, and resin; and a hole for injecting the fluid sample into the flow path and a hole for draining the flowed fluid sample therefrom which are opened into the substrate sheet;
a pressurizer, which is connected to the hole for injecting the fluid sample, for flowing the fluid sample into the flow path by pressing it after injecting it; and
a device body for installing the microchemical chip.
